# EUROPÄISCHE PATENTSCHRIFT

(11) **EP 2 796 447 B1**
(45) Veröffentlichungstag und Bekanntmachung des Hinweises auf die Patenterteilung: **30.03.2016**
(21) Anmeldenummer: 13165029.3
(22) Anmeldetag: 24.04.2013
(51) Int. Cl.: C07C 323/52, C10M 135/06, C10M 135/26

(54) **Neue schwefelbrückenhaltige Verbindungen, Verfahren zu deren Herstellung und deren Verwendung**
New compounds containing sulfide bridges, method for their manufacture and use thereof
Nouveaux composés contenant des ponts de soufre, leur procédé de fabrication et leur utilisation

(43) Veröffentlichungstag der Anmeldung: 29.10.2014
(73) Patentinhaber: Rhein Chemie Rheinau GmbH, 68219 Mannheim (DE)
(72) Erfinder: Larem, David, 64859 Eppertshausen (DE); Horstmann, Sandra, 68167 Mannheim (DE); Rossrucker, Thomas, 76684 Östringen (DE); Kuilder, Markus, 68723 Oftersheim (DE); König, Michael, 68239 Mannheim (DE)
(74) Vertreter: Siegers, Britta

(56) Entgegenhaltungen:
- EP-A1- 0 009 701
- US-A- 3 822 299
- US-A- 4 172 800

## Beschreibung

Die vorliegende Erfindung betrifft neue schwefelbrückenhaltige Verbindungen, bei denen innerhalb des Moleküls mindestens eine Fettsäure über mindestens eine Schwefelbrücke an mindestens einen Polyalkylenglykolester, dem Umsetzungsprodukt aus einer Fettsäure mit einem Polyalkylenglykol, verbunden ist, diese einen Schwefelgehalt von 8 bis 29 Gew.-% aufweisen, Verfahren zu deren Herstellung und deren Verwendung als Schwefelträger und Schmierstoffadditiv.

In Lubricant Additives, 2003, S. 259 und S. 260 wird beschrieben, dass Schwefelträger eine Klasse von organischen Verbindungen sind, worin der Schwefel eine Oxidationsstufe von 0 oder -1 aufweist und das Schwefelatom entweder an einen Kohlenwasserstoff oder an ein Schwefelatom gebunden ist. Weiterhin wird erklärt, dass Schwefelträger keine weiteren Heteroatome außer Sauerstoff besitzen. Die Herstellung der Schwefelträger erfolgt mittels Zugabe von Schwefel zu allen Arten von ungesättigten Verbindungen, welche Doppelbindungen enthalten.

Schwefelträger werden hauptsächlich als Additiv in Schmierstoffen verwendet. Gemäß der üblichen Einteilung der Schmierstoffadditive werden Schwefelträger zur Gruppe der Hochdruckadditive (= EP-Additive) gerechnet, was in ihrer vorrangigen Wirkungsweise begründet ist. Vornehmlich verhindern Schwefelträger das Verschweißen von zwei aneinander reibenden metallischen Werkstoffen, und zwar bei hoher Belastung und hoher Temperatur. In Lubricants and Lubrications, 2001, S. 107 wird beschrieben, dass der enthaltene Schwefel mit der Metalloberfläche reagiert, wobei diese Reaktion im Allgemeinen bei Temperaturen von über 600 °C stattfindet. Die resultierenden gleitfähigen Reaktionsschichten werden mittels einer Art von kontrolliertem Verschleiß kontinuierlich abgeschert, wodurch das Verschweißen der Metalloberflächen verhindert wird.

Zudem mindern Schwefelträger die Reibung von zwei aneinander reibenden metallischen Werkstoffen, wobei diese Wirkungsweise hauptsächlich bei niederer Belastung und niederer Temperatur von Bedeutung ist. Zur Minderung der Reibung ist die Schmierleistung des Schwefelträgers maßgeblich. In Lubricant Additives, 2003, S. 277 wird beschrieben, dass die Schmierleistung von Schwefelträgern mit zunehmender Polarität im Allgemeinen steigt.

Schwefelträger, welche aus schwefelverbrückten Triglyceriden und/oder Fettsäurealkylestern bestehen, werden mit ungesättigten Triglyceriden und/oder ungesättigten Fettsäurealkylestern hergestellt, welche anschließend schwefelverbrückt werden. Schwefelverbrückte Triglyceride und/oder Fettsäurealkylester haben den Nachteil, dass sie nicht wassermischbar sind. Bei deren Verwendung in wassermischbaren Schmierstoffen müssen deshalb Emulgatoren zugesetzt werden.

Schwefelträger, welche aus schwefelverbrückten Fettsäuren bestehen, werden mit ungesättigten Fettsäuren hergestellt, welche schwefelverbrückt werden. Die schwefelverbrückten Fettsäuren werden vornehmlich als Additiv für wassermischbare Schmierstoffe verwendet. Bei der Herstellung von wassermischbaren Schmierstoffen werden die schwefelverbrückten Fettsäuren normalerweise zu Seifen neutralisiert, weil standardmäßig Basen, wie z.B. Kalilauge oder Triethanolamin, zur Einstellung eines alkalischen pH-Werts zugesetzt werden. Die dann vorliegenden schwefelverbrückten Seifen sind wassermischbar und benötigen keinen Emulgatorzusatz, sondern können selbst als Emulgator wirken. Weiterhin sind sie stark polar und besitzen eine hohe Schmierleistung auf metallischen Werkstoffen. Schwefelverbrückte Seifen haben jedoch den Nachteil, dass sie Kalkseifenabscheidungen in hartem Wasser hervorrufen können, wie z. B. beschrieben in Lubricant Additives, 2003, S. 281. Verschiedene Lösungsansätze, wie z. B. die Anpassung der Schmierstoff-Formulierung auf einen vorgegebenen Wasserhärte-Bereich oder der Einsatz von Komplexbildnern, zeigten sich bisher nur als Hilfslösung. In der Regel muss man einen hohen Aufwand leisten, um schwefelverbrückte Fettsäuren als Additiv für wassermischbare Schmierstoffe zufriedenstellend verwenden zu können. Dadurch ist deren Einsatz stark begrenzt.

In US-A 4172800 und US-A 3822299 werden ungesättigte Fettsäuren zunächst ethoxyliert und anschließend mit Schwefel zu schwefelverbrückten Fettsäurepolyalkylenglykolestern umgesetzt. Die beschriebenen Produkte verfügen über keine Carbonsäureeinheit. Diese Produkte sind nicht wirtschaftlich einsetzbar wegen eines niedrigen Schwefelgehaltes von weniger als 8 Gew.-%, und/oder sie sind nicht vollständig wassermischbar. In US-A 3822299 wird im Beispiel 2 ein vergleichsweise hoher Schwefelgehalt von 10,9 Gew.-% erreicht. Die Berechnung des HLB-Wertes nach W. C. Griffin ergibt einen HLB-Wert von 7,5. Demnach bildet das Produkt aus Beispiel 2 in Wasser verdünnt eine semistabile Emulsion und ist somit nicht vollständig wassermischbar.

Alle bisher bekannten Schwefelträger erfordern die Zugabe von Emulgatoren zur vollständigen Mischbarkeit in Wasser und/oder besitzen eine schwach ausgeprägte Polarität und/oder haben die Neigung zur Bildung von Kalkseifenabscheidungen in hartem Wasser und/oder haben einen niedrigen Schwefelgehalt. Hartes Wasser im Sinne der Erfindung bedeutet einen Härtegrad von wenigstens 8,4 °dH. Dies entspricht den Härtebereichen "mittel" und "hart" gemäß dem deutschen Wasch- und Reinigungsmittelgesetz aus dem Jahre 2007.

Die Aufgabe der vorliegenden Erfindung bestand somit darin, schwefelbrückenhaltige Verbindungen bereitzustellen, welche ohne Zugabe von Emulgatoren vollständig wassermischbar sind, zudem eine starke Polarität besitzen, keine oder keine maßgebliche Neigung zur Bildung von Kalkseifenabscheidungen in hartem Wasser haben und über einen Schwefelgehalt von wenigstens 8 Gew.-% verfügen.

Überraschenderweise wurde nun gefunden, dass schwefelbrückenhaltige Verbindungen, bei denen innerhalb des Moleküls mindestens eine Fettsäure über mindestens eine Schwefelbrücke an mindestens einen Polyalkylenglykolester, dem Umsetzungsprodukt aus einer Fettsäure mit einem Polyalkylenglykol, verbunden ist und diese durch die Schwefelbrücke(n) einen Schwefelgehalt von 8 bis 29 Gew.-% aufweisen, diese Aufgabe erfüllen.

Gegenstand der vorliegenden Erfindung sind schwefelbrückenhaltige Verbindungen, bei denen innerhalb des Moleküls mindestens eine Fettsäure über mindestens eine Schwefelbrücke an mindestens einen Polyalkylenglykolester, dem Umsetzungsprodukt aus einer Fettsäure mit einem Polyalkylenglykol, verbunden ist und diese durch die Schwefelbrücke(n) einen Schwefelgehalt von 8 bis 29 Gew.-% aufweisen.

Bei dem Polyalkylenglykolester handelt es sich vorzugsweise um Polyethylenglykolester, Polypropylenglykolester, Polybutylenglykolester und/oder um Polyalkylenglykolester, bei denen die Ethylenoxid-, Propylenoxid- und/oder Butylenoxid-Einheiten statistisch und/oder blockweise angeordnet sind. Besonders bevorzugt ist Polyethylenglykolester. Im weiteren Text werden die Polyalkylenglykolester auch als mit Polyalkylenglykol veresterte Fettsäuren bezeichnet.

Die erfindungsgemäßen schwefelbrückenhaltigen Verbindungen auf Basis von monofunktionellem Polyethylenglykol und einfach ungesättigten Monocarbonsäuren lassen sich schematisch mit folgender Formel (I) darstellen:

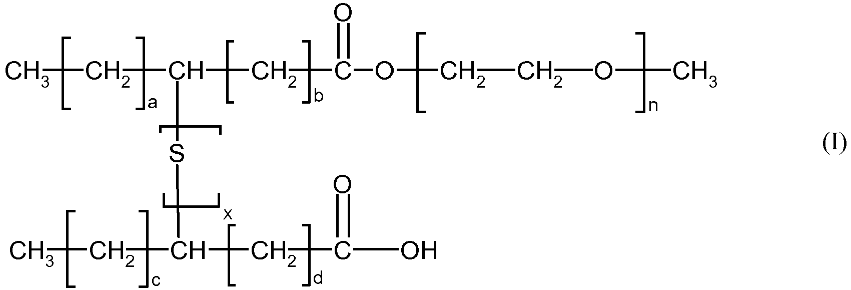

Die erfindungsgemäßen schwefelbrückenhaltigen Verbindungen auf Basis von bifunktionelle Polyethylenglykol und einfach ungesättigten Monocarbonsäuren lassen sich schematisch mit folgender Formel (II) darstellen:

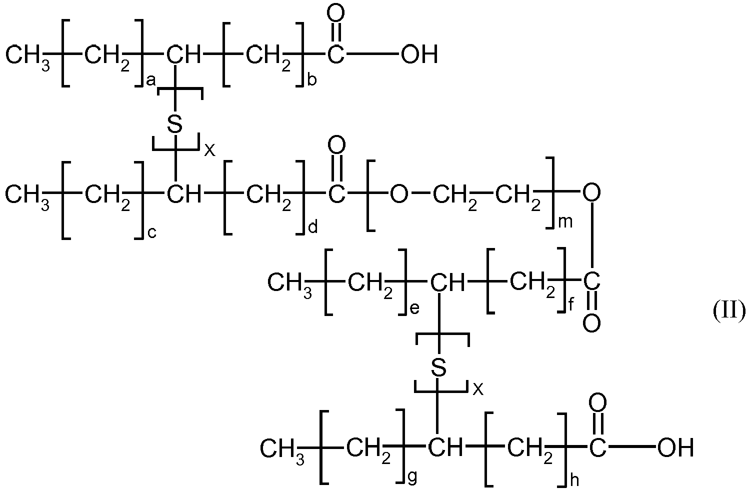

Die erfindungsgemäßen schwefelbrückenhaltigen Verbindungen auf Basis von trifunktionellem Polyethylenglykol und einfach ungesättigten Monocarbonsäuren lassen sich schematisch mit folgender Formel (III) darstellen:

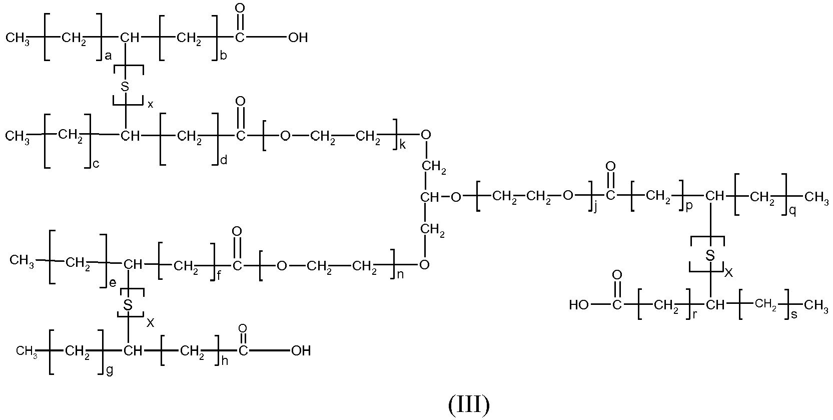

Die erfindungsgemäßen schwefelbrückenhaltigen Verbindungen auf Basis von tetrafunktionellem Polyethylenglykol und einfach ungesättigten Monocarbonsäuren lassen sich schematisch mit folgender Formel IV darstellen:

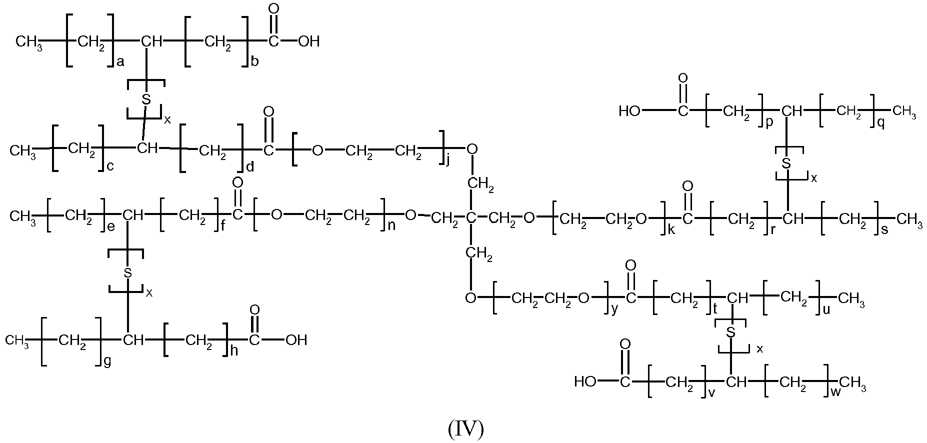

Für die angegebenen Indizes sind die folgenden Zahlenbereiche bevorzugt:

| **Index** | **Zahlenbereich** | **Bevorzugter Zahlenbereich** |
|---|---|---|
| a | 3 bis 29 | 5 bis 12 |
| b | 3 bis 29 | 5 bis 12 |
| c | 3 bis 29 | 5 bis 12 |
| d | 3 bis 29 | 5 bis 12 |
| e | 3 bis 29 | 5 bis 12 |
| f | 3 bis 29 | 5 bis 12 |
| g | 3 bis 29 | 5 bis 12 |
| h | 3 bis 29 | 5 bis 12 |
| p | 3 bis 29 | 5 bis 12 |
| q | 3 bis 29 | 5 bis 12 |
| r | 3 bis 29 | 5 bis 12 |
| s | 3 bis 29 | 5 bis 12 |
| t | 3 bis 29 | 5 bis 12 |
| u | 3 bis 29 | 5 bis 12 |
| v | 3 bis 29 | 5 bis 12 |
| w | 3 bis 29 | 5 bis 12 |
| n | 2 bis 10 | 4 bis 8 |
| m | 4 bis 20 | 8 bis 16 |
| k | 2 bis 10 | 4 bis 8 |
| j | 2 bis 10 | 4 bis 8 |
| x | 1 bis 10 | 2 bis 4 |
| y | 2 bis 10 | 4 bis 8 |

Die Formeln II, III und IV können noch weitere Bausteine, wie Polyethylenglykolester, Schwefelbrücken und/oder gesättigte Monocarbonsäuren enthalten. Die in allen Formeln dargestellten schwefelbrückenhaltigen Verbindungen basieren auf Polyethylenglykolen. Von der Erfindung umfasst sind neben den Polyethylenglykolen auch Polypropylenglykole, Polybutylenglykole und/oder Polyalkylenglykole, bei denen die Ethylenoxid-, Propylenoxid- und /oder Butylenoxid-Einheiten statistisch und/oder blockweise angeordnet sind. Weiterhin können die monofunktionellen, bifunktionellen, trifunktionellen und tetrafunktionellen Polyalkylenglykole als Gemisch eingesetzt werden. Neben den einfach ungesättigten Monocarbonsäuren können auch ungesättigte Fettsäuren zum Einsatz kommen, welche eine beliebige Anzahl an Doppelbindungen, Dreifachbindungen und/oder Carbonsäureeinheiten besitzen.

Die Herstellung der erfindungsgemäßen schwefelbrückenhaltigen Verbindungen kann über die nachstehend genannten 3 Ausführungsformen erfolgen, wobei die erste Ausführungsform bevorzugt ist:
1) Die schwefelbrückenhaltigen Verbindungen sind erhältlich durch die Umsetzung der ungesättigten Fettsäuren mit Schwefel und/oder Schwefelwasserstoff zu schwefelverbrückten Fettsäuren, vorzugsweise schwefelverbrückten gesättigten Fettsäuren, und die anschließende Umsetzung dieser schwefelverbrückten Fettsäuren mit Polyalkylenglykol.
2) Die schwefelbrückenhaltigen Verbindungen sind erhältlich durch die Umsetzung der ungesättigten Fettsäuren mit Polyalkylenglykol zum Zwischenprodukt, bestehend aus ungesättigtem Polyalkylenglkyolester und unveresterter ungesättigter Fettsäure, und die anschließende Umsetzung dieses Zwischenprodukts mit Schwefel und/oder Schwefelwasserstoff.
3) Die schwefelbrückenhaltigen Verbindungen sind erhältlich durch das Vermischen der ungesättigten Fettsäuren mit ungesättigten Fettsäurepolyalkylenglykolestern, die aus einer Umsetzung von ungesättigten Fettsäuren mit Polyalkylenglykol oder Alkylenoxiden resultieren, und die anschließende Umsetzung dieses Gemisches mit Schwefel und/oder Schwefelwasserstoff.

Im Sinne dieser Erfindung handelt es sich bei Polyalkylenglykol um monofunktionelles, bifunktionelles, trifunktionelles und/oder tetrafunktionelles Polyalkylenglykol.

Bei den monofunktionellen Polyalkylenglykolen handelt es sich vorzugsweise um lineare Polyalkylenglykole, welche eine Hydroxygruppe besitzen, wobei Alkylen vorzugsweise für Ethylen, Propylen und/oder Butylen, besonders bevorzugt Ethylen steht. Die Molmasse der monofunktionellen Polyalkylenglykole beträgt vorzugsweise 100 bis 500 g/mol. Diese sind herstellbar durch die Umsetzung von monofunktionellen Alkoholen, vorzugsweise Methanol, mit Alkylenoxiden. Die monofunktionellen Polyalkylenglykole sind z.B. bei der Firma Clariant International AG unter dem Handelsnamen Polyglykol M erhältlich.

Bei den bifunktionellen Polyalkylenglykolen handelt es sich vorzugsweise um lineare Polyalkylenglykole, welche zwei Hydroxygruppen besitzen, wobei Alkylen vorzugsweise für Ethylen, Propylen und/oder Butylen, besonders bevorzugt Ethylen steht. Die Molmasse der bifunktionellen Polyalkylenglykole beträgt vorzugsweise 200 bis 1000 g/mol. Diese sind herstellbar durch die Umsetzung von bifunktionellen Alkoholen, vorzugsweise Ethylenglykol oder Diethylenglykol, mit Alkylenoxiden. Die bifunktionellen Polyalkylenglykole sind z.B. bei der Firma BASF SE unter dem Handelsnamen Pluriol® E erhältlich.

Bei den trifunktionellen Polyalkylenglykolen handelt es sich vorzugsweise um verzweigte Polyalkylenglykole, welche drei Hydroxygruppen besitzen, wobei Alkylen vorzugsweise für Ethylen, Propylen und/oder Butylen, besonders bevorzugt Ethylen steht. Die Molmasse der trifunktionellen Polyalkylenglykole beträgt vorzugsweise 300 bis 1500 g/mol. Diese sind herstellbar durch die Umsetzung von trifuktionellen Alkoholen, vorzugsweise Glycerin, mit Alkylenoxiden. Die trifunktionellen Polyalkylenglykole sind z.B. bei der Firma The Dow Chemical Company unter dem Handelsnamen Ucon™ TPEG erhältlich.

Bei den tetrafunktionellen Polyalkylenglykolen handelt es sich vorzugsweise um verzweigte Polyalkylenglykole, welche vier Hydroxygruppen besitzen, wobei Alkylen vorzugsweise für Ethylen, Propylen und/oder Butylen, besonders bevorzugt Ethylen steht. Die Molmasse der tetrafunktionellen Polyalkylenglykole beträgt vorzugsweise 400 bis 2000 g/mol. Diese sind herstellbar durch die Umsetzung von tetrafunktionellen Alkoholen, vorzugsweise Pentaerythrit, mit Alkylenoxiden. Die tetrafunktionellen Polyalkylenglykole sind z.B. bei der Firma Clariant International AG unter dem Handelsnamen Polyglykol P41 erhältlich.

Bei den vorstehend genannten Alkylenoxiden sind Ethylenoxid, Propylenoxid und/oder Butylenoxid bevorzugt. Diese sind z. B. bei der Firma BASF SE kommerziell erhältlich.

Die allgemeine Herstellung von Polyalkylenglykolen ist beschrieben in Lubricants and Lubrication, 2001, S. 75.

Bei der mindestens einen Fettsäure und der mindestens einen mit Polyalkylenglykol veresterten Fettsäure (Polyalkylenglykolester) handelt es sich vorzugsweise um gesättigte Carbonsäuren, vorzugsweise gesättigte Monocarbonsäuren, die vorzugsweise eine Kohlenstoffkettenlänge von 6 bis 32 C-Atomen haben. Als Edukte können sowohl reine ungesättigte Monocarbonsäuren und/oder Gemische von Monocarbonsäuren, welche als Hauptbestandteile ungesättigte Monocarbonsäuren und als Nebenbestandteile gesättigte Monocarbonsäuren enthalten, eingesetzt werden.

Für die Reaktion mit Schwefel beziehungsweise Polyalkylenglykol werden ungesättigte Fettsäuren, vorzugsweise ungesättigte Monocarbonsäuren, eingesetzt. Bevorzugt sind dabei Ölsäure und Linolsäure, die in einer besonders bevorzugten Ausführungsform der Erfindung als Hauptbestandteile eines Fettsäuregemisches eingesetzt werden.

Die Fettsäuregemische werden vorzugsweise aus Ölen von natürlichem Ursprung gewonnen und können neben den ungesättigten Fettsäuren auch einen Anteil an gesättigten Fettsäuren enthalten. Öle von natürlichem Ursprung sind z. B. Babassuöl, Baumwollsaatöl, Borretschöl, Distelöl (= Safloröl), Erdnussöl, Johannisbeerkernöl, Haselnussöl, Heringsöl, Holzöl, Jojobaöl, Kokosöl, Klauenöl, Knochenöl, Lardöl (= Schweineschmalz), Leberöl, Leinöl, Maiskeimöl, Mandelöl, Olivenöl, Palmöl, Palmkernöl, Rapsöl (= Rüböl), Rindertalgöl (= Rinderschmalz), Rizinusöl, Sardinenöl, Senfsaatöl, Sojabohnenöl, Sonnenblumenöl, Sheabutter, Tallöl, Traubenkernöl, Walöl, Walnussöl. Bevorzugt eingesetzt werden deren raffinierte Varianten. Dabei sind solche Fettsäuregemische bevorzugt, welche mehr als 80 Gew.-% ungesättigte Fettsäuren beinhalten.

Die ungesättigten Fettsäurepolyalkylenglykolester, welche gemäß der dritten Ausführungsform als Edukt verwendet werden, werden durch die Reaktion von ungesättigten Fettsäuren mit Polyalkylenglykolen oder Alkylenoxiden hergestellt. Dabei handelt es sich um kommerziell erhältliche Produkte, die aber auch wie vorgenannt, durch die Umsetzung von ungesättigten Fettsäuren mit Polyalkylenglykolen oder Alkylenoxiden hergestellt werden können. In diesem Zusammenhang wird in Bezug auf die bevorzugten ungesättigten Fettsäuren, Polyalkylenglykole und Alkylenoxide auf die vorhergehenden Ausführungen verwiesen. Jene Reaktion von ungesättigten Fettsäuren mit Polyalkylenglykolen wird vorzugsweise bei Temperaturen von 120 bis 200 °C und bei Drücken von 20 mbar bis Normaldruck und bei einer Reaktionszeit von 4 bis 24 h unter Einsatz eines sauren Katalysators durchgeführt. Die Reaktion von ungesättigten Fettsäuren mit Alkylenoxiden wird vorzugsweise bei einer Temperatur von 100 bis 190 °C und einem Druck von 1 bis 6 bar unter Einsatz eines nucleophilen Katalysators durchgeführt. Die ungesättigten Fettsäurepolyalkylenglykolester sind z.B. bei der Firma Sasol Germany GmbH unter den Handelsnamen Marlosol® und Marlowet® erhältlich.

Die allgemeine Herstellung von Fettsäurepolyalkylenglykolestern ist beschrieben in Chemistry and Technology of Surfactants, 2006, S. 139 und S. 140.

Die erfindungsgemäßen schwefelbrückenhaltigen Verbindungen können auch Bestandteil einer Mischung sein.

Ein weiterer Gegenstand der Erfindung sind Mischungen enthaltend diese erfindungsgemäßen schwefelbrückenhaltige Verbindungen und zusätzlich weitere Additive und/oder Basisflüssigkeiten.

Dabei bevorzugt sind weitere Hochdruck-Additive, Schwefeladditive, Verschleißschutz-Additive, Phosphoradditive, Korrosionsinhibitoren, Sulfonate, Sulfonsäuren, Sulfonsäureester, Carboxylate, Carbonsäuren, Carbonsäureester, Buntmetalldesaktivatoren, Triazole, Triazolderivate, Tenside, Emulgatoren, Dispergatoren, Lösungsvermittler, Carbonsäurealkoxylate, Carbonsäureamide, Fettalkohole, Fettalkoholalkoxylate, Ethercarbonsäuren, Glykole, Glykolether, Polyalkylen-glykole, Alkalisatoren, Alkylamine, Alkanolamine, Schmierfähigkeitsverbesserer, Monoglyceride, Diglyceride, Triglyceride, Biozide, Entschäumer, Antioxidantien, Komplexbildner, Sequestriermittel, Demulgatoren, Viskositätsindexverbesserer, Flammschutzmittel, Farbstoffe, Duftstoffe sowie Group I bis V Öle gemäß der Definition des American Petroleum Institute (API).

Gegenstand der vorliegenden Erfindung sind zudem Verfahren zur Herstellung der erfindungsgemäßen schwefelbrückenhaltigen Verbindungen:
Dabei sind die 3 nachstehend genannten Ausführungsformen bevorzugt.
   1) Die ungesättigte Fettsäure, vorzugsweise die ungesättigte Monocarbonsäure, wird zunächst mit Schwefel und/oder Schwefelwasserstoff zur schwefelverbrückten Fettsäure, vorzugsweise schwefelverbrückten gesättigten Fettsäure, umgesetzt, und anschließend erfolgt die Umsetzung dieser schwefelverbrückten Fettsäure mit mindestens einem Polyalkylenglykol, so dass innerhalb der erfindungsgemäßen schwefelbrückenhaltigen Verbindungen mindestens eine Carbonsäureeinheit der Fettsäure enthalten ist.
   2) Ein molarer Überschuss an ungesättigter Fettsäure, vorzugsweise an ungesättigter Monocarbonsäure, wird mit mindestens einem Polyalkylenglykol zum Zwischenprodukt, bestehend aus ungesättigtem Polyalkylenglkyolester und unveresterter ungesättgter Fettsäure, umgesetzt, und anschließend erfolgt die Umsetzung dieses Zwischenprodukts mit Schwefel und/oder Schwefelwasserstoff, so dass innerhalb der erfindungsgemäßen schwefelbrückenhaltigen Verbindungen mindestens eine Carbonsäureeinheit der Fettsäure enthalten ist.
   3) Die ungesättigte Fettsäure, vorzugsweise die ungesättigte Monocarbonsäure, wird mit mindestens einem ungesättigten Fettsäurepolyalkylenglykolester gemischt, und anschließend erfolgt die Umsetzung dieses Gemisches mit Schwefel und/oder Schwefelwasserstoff, so dass innerhalb der erfindungsgemäßen schwefelbrückenhaltigen Verbindungen mindestens eine Carbonsäureeinheit der Fettsäure enthalten ist.

Die Bestimmung der Carbonsäureeinheit erfolgt über die Säurezahl gemäß ASTM D-664 beziehungsweise DIN 53402. Bei der ersten und zweiten Ausführungsform des erfindungsgemäßen Verfahrens wird die Umsetzung der Fettsäure und/oder schwefelverbrückten Fettsäure mit dem Polyalkylenglykol durch die Abnahme der Säurezahl nachgewiesen. Die Umsetzung wird spätestens dann beendet, wenn noch mindestens eine Carbonsäureeinheit vorhanden ist.

Bei der ersten und zweiten Ausführungsform des erfindungsgemäßen Verfahrens werden die Edukte vorzugsweise in folgenden Verhältnissen eingesetzt:
a) Bezogen auf 1 g ungesättigter Fettsäure, vorzugsweise ungesättigter Monocarbonsäure, wird 0,05 g bis 1 g Polyalkylenglykol eingesetzt;
   sowie
b) bezogen auf 1 g ungesättigter Fettsäure, vorzugsweise ungesättigter Monocarbonsäure, wird 0,05 g bis 0,5 g Schwefel eingesetzt.

Bei der dritten Ausführungsform des erfindungsgemäßen Verfahrens werden die Edukte vorzugsweise in folgenden Verhältnissen eingesetzt:
a) Bezogen auf 1 g ungesättigter Fettsäure, vorzugsweise ungesättigter Monocarbonsäure, wird 0,2 g bis 4 g ungesättigter Fettsäurepolyalkylenglykolester eingesetzt;
   sowie
b) bezogen auf 1 g ungesättigter Fettsäure, vorzugsweise ungesättigter Monocarbonsäure, wird 0,05 g bis 0,5 g Schwefel eingesetzt.

Bei allen Ausführungsformen der erfindungsgemäßen Verfahren wird die Umsetzung mit Schwefel und/oder Schwefelwasserstoff ("Schwefelung") vorzugsweise bei Drücken von Normaldruck (d. h. in einem Bereich von 0,9 bis 1,1 bar) bis 15 bar und bei Temperaturen von 119 bis 170 °C und bei einer Reaktionszeit von 4 bis 24 h durchgeführt.

Bei der ersten und zweiten Ausführungsform des erfindungsgemäßen Verfahrens wird die Veresterung vorzugsweise bei Normaldruck (d. h. in einem Bereich von 0,9 bis 1,1 bar) gestartet, danach auf Drücke bis zu 10 mbar reduziert und bei Temperaturen von 120 bis 200 °C und bei einer Reaktionszeit von 4 bis 24 h durchgeführt.

Bei der dritten Ausführungsform des erfindungsgemäßen Verfahrens wird das Vermischen vorzugsweise bei Normaldruck und bei Temperaturen von 15 bis 100 °C und bei einer Mischzeit von 5 bis 30 min durchgeführt.

In weiteren Ausführungsformen der vorliegenden Erfindung sind die nachstehend genannten Reaktionsbedingungen bevorzugt:
In der ersten Ausführungsform des erfindungsgemäßen Verfahrens wird vorzugsweise die ungesättigte Fettsäure, vorzugsweise die ungesättigte Monocarbonsäure, zunächst mit Schwefel und/oder Schwefelwasserstoff und anschließend mit Polyalkylenglykol, vorzugsweise Polyethylenglykol, zu den erfindungsgemäßen schwefelbrückenhaltigen Verbindungen umgesetzt. Im ersten Schritt wird die ungesättigte Monocarbonsäure, mit Schwefel und/oder Schwefelwasserstoff in einem Druckreaktor bei Normaldruck bis 15 bar, vorzugsweise 4 bar, und bei 119 bis 170 °C, vorzugsweise 130 °C, geschwefelt. Die Schwefelung wird vorzugsweise durch Amine, Metalloxide oder Säuren katalysiert. Im zweiten Schritt wird das Zwischenprodukt mit Polyalkylenglykol, vorzugsweise Polyethylenglykol, bei Normaldruck bis 10 mbar und bei 120 bis 200 °C, bevorzugt 180 °C, verestert. Die Veresterung wird vorzugsweise solange durchgeführt, bis kein Wasser mehr abdestilliert wird. Die Veresterung wird vorzugsweise durch Zinnsalze oder Säuren, vorzugsweise durch Phosphorsäure und/oder p-Toluolsulfonsäure, katalysiert.

In der zweiten Ausführungsform des erfindungsgemäßen Verfahrens wird vorzugsweise ein molarer Überschuss an ungesättigter Fettsäure, vorzugsweise die ungesättigte Monocarbonsäure, zunächst mit Polyalkylenglykol, vorzugsweise Polyethylenglykol, und anschließend mit Schwefel und/oder Schwefelwasserstoff zu den erfindungsgemäßen schwefelbrückenhaltigen Verbindungen umgesetzt. Im ersten Schritt wird die ungesättigte Monocarbonsäure mit Polyalkylenglykol, vorzugsweise Polyethylenglykol, bei Normaldruck bis 10 mbar und bei 120 bis 200 °C, bevorzugt 180 °C, verestert. Die Veresterung wird vorzugsweise solange durchgeführt, bis kein Wasser mehr abdestilliert wird. Die Veresterung wird vorzugsweise durch Zinnsalze oder Säuren, vorzugsweise durch Phosphorsäure oder p-Toluolsulfonsäure, katalysiert. Im zweiten Schritt wird das Zwischenprodukt, welches ungesättigten Polyalkylenglkyolester und unveresterte ungesättgte Fettsäure enthält, mit Schwefel und/oder Schwefelwasserstoff in einem Druckreaktor bei Normaldruck bis 15 bar, vorzugsweise 4 bar, und bei 119 bis 170 °C, vorzugsweise 130 °C, geschwefelt. Die Schwefelung wird vorzugsweise durch Amine, Metalloxide oder Säuren katalysiert.

In der dritten Ausführungsform des erfindungsgemäßen Verfahrens wird die ungesättigte Fettsäure, vorzugsweise die ungesättigte Monocarbonsäure, zunächst mit dem ungesättigten Fettsäurepolyalkylenglykolester gemischt, und anschließend mit Schwefel und/oder Schwefelwasserstoff zu den erfindungsgemäßen schwefelbrückenhaltigen Verbindungen umgesetzt. Im ersten Schritt wird die ungesättigte Monocarbonsäure mit den ungesättigten Fettsäurepolyalyklenglykolestern bei Normaldruck und bei 15 bis 100 °C, bevorzugt 40 °C, gemischt. Im zweiten Schritt wird das Gemisch mit Schwefel und/oder Schwefelwasserstoff in einem Druckreaktor bei Normaldruck bis 15 bar, vorzugsweise 4 bar, und bei 120 bis 170 °C, vorzugsweise 130 °C, geschwefelt. Die Schwefelung wird vorzugsweise durch Amine, Metalloxide oder Säuren katalysiert.

Gegenstand der vorliegenden Erfindung ist zudem ein Verfahren zur Herstellung einer Mischung, wonach zusätzlich Schmierstoffadditive und Basisflüssigkeiten vor, während oder nach der Herstellung der erfindungsgemäßen schwefelbrückenhaltigen Verbindungen zugesetzt werden.

Für die Zudosierung sind handelsübliche Mischaggregate, vorzugsweise Rührkessel, einsetzbar.

Gegenstand der vorliegenden Erfindung ist zudem die Verwendung der erfindungsgemäßen schwefelbrückenhaltigen Verbindungen als Schwefelträger und/oder als Schmierstoffadditiv in sämtlichen Schmierstoffen gemäß ISO 6743, wie vorzugsweise Schmierstoffe für Metallbearbeitungen oder Schmierstoffe für Maschinen.

Die erfindungsgemäßen schwefelbrückenhaltigen Verbindungen werden vorzugsweise als Schmierstoffadditiv in wassermischbaren oder wassergemischten Kühlschmierstoffen eingesetzt. Diese sind beschrieben in DIN 51385.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Die nachfolgenden Beispiele dienen der Erläuterung der Erfindung, ohne dabei limitierend zu wirken.

### Ausführungsbeispiele:

In dem nachfolgenden Beispiel betreffen die Prozentangaben Gew.-%.

### Reagenzien:

Additin® RC 5250, schwefelverbrückte Fettsäuren auf Basis eines pflanzliches Fettsäuregemisches (bestehend aus: > 90 Gew.-% ungesättigten Fettsäuren, Hauptbestandteile Ölsäure und Linolsäure), mit einem Schwefelgehalt von ca. 15 Gew.-%; Hersteller: Firma Rhein Chemie Rheinau GmbH.

Pluriol® E 600, lineares bifunktionelles Polyethylenglykol, mittlere Molmasse: ca. 600 g/mol; Hersteller: Firma BASF SE.

Die folgenden Komponenten wurden mittels Magnetrührer bei etwa 25 °C gemischt (Mischung A1):
20,00 g Additin® RC 5250
10,00 g Pluriol® E 600
0,03 g Phosphorsäure, 75 Gew.-%
Die Säurezahl betrug 115,9 mg KOH/g.
Die Versuche wurden wie folgt durchgeführt:

Von der Mischung A1 wurden 5 Tropfen entnommen und bei etwa 25 °C in etwa 50 ml Leitungswasser mit einer Wasserhärte von 20 °dH gegeben (Probe A1). Nach dem Umrühren der Probe A1 verblieben im Leitungswasser unlösliche Tropfen.

Danach wurden 20 g von der Mischung A1 mittels Magnetrührer bei 120 °C im offenen Becherglas erhitzt. Nach einer Reaktionszeit von 6 Stunden wurde das Reaktionsprodukt, d.h. eine Mischung aus den erfindungsgemäßen schwefelbrückenhaltigen Verbindungen, gegebenenfalls nicht umgesetzten Edukten und Nebenprodukten, auf Raumtemperatur abgekühlt (Mischung A2). Von der Mischung A2 wurden ebenfalls 5 Tropfen entnommen und in etwa 50 ml Leitungswasser mit einer Wasserhärte von 20 °dH gegeben (Probe A2). Nach dem Umrühren der Probe A2 wurden die Tropfen in Wasser emulgiert und bildeten eine grobdisperse Emulsion.

Abschließend wurden zu den Proben A1 und A2 jeweils einige Tropfen Kalilauge gegeben, bis ein pH-Wert von > 10 erreicht wurde. In der neutralisierten Probe A1 bildeten sich voluminöse Abscheidungen und in der neutralisierten Probe A2 bildete sich eine feindisperse Emulsion, die erst nach einigen Tagen einen schwachen, feinen Bodensatz aufwies.

In der nachfolgenden Tabelle sind die Versuchsergebnisse zusammengefasst:

| | **Mischung A1 (Vergleich)** | **Mischung A2 (erfindungsgemäß)** |
|---|---|---|
| **Löslichkeit in Leitungswasser** | Unlöslich | Grobdisperse Emulsion |
| **Löslichkeit in Leitungswasser nach Neutralisation** | Voluminöse Abscheidungen | Feindisperse Emulsion |
| **Gemessene Säurezahl nach 6 Stunden** | 115,9 mg KOH/g | 85,4 mg KOH/g |
| **Korrigierte Säurezahl (= gemessene Säurezahl abzüglich der berechneten Säurezahl des Phosphorsäure-Anteils)** | 114,6 mg KOH/g | 84,1 mg KOH/g |
| **Gemessener Schwefelgehalt** | 10,3 Gew.-% | 10,3 Gew.-% |

Die Mischung A1 wurde nach einer Reaktionszeit von 6 Stunden zum erfindungsgemäßen Produkt (Mischung A2) umgesetzt. Die erfindungsgemäßen schwefelbrückenhaltigen Verbindungen sind schwefelverbrückt und haben einen Schwefelgehalt von 10,3 Gew.-%. Die Säurezahl des erfindungsgemäßen Produkts beträgt 85,4 mg KOH/g. Aufgrund der Abnahme der Säurezahl wurde der Nachweis erbracht, dass innerhalb der schwefelbrückenhaltigen Verbindungen sowohl unveresterte Fettsäuren als auch mit Polyethylenglykol veresterte Fettsäuren (Polyethylenglykolester) vorhanden sind. Der Wert für die Mischung A1 (Vergleich) blieb unverändert.

Weiterhin wurden quantitative Untersuchungen zur Bestimmung von Kalkseifenabscheidungen durchgeführt. Hierzu wurden die Mischungen A1 und A2 bei etwa 25 °C jeweils zu 0,3 Gew.-% in Wasser gemäß DIN 51367 mit einer definierten Gesamthärte von 3,58 mmol/Liter, dies entspricht 20 °dH, gelöst. Diese Lösungen wurden jeweils mit Kalilauge (45 Gew.-%ig) versetzt, bis ein pH-Wert von 11,0 ± 0,2 erreicht wurde. Um die Mischungen A1 und A2 leichter in Wasser zu lösen, wurde der größere Teil der benötigten Kalilauge zuerst in Wasser gelöst. Während stetigem Rühren wurden dann die Mischungen A1 und A2 jeweils langsam zugetropft. Abschließend wurde mit der Kalilauge ein pH-Wert von 11,0 ± 0,2 eingestellt.

Die 0,3 gewichtsprozentige Lösung von der Mischung A1 wird als Lösung A1 bezeichnet und die 0,3 gewichtsprozentige Lösung von der Mischung A2 wird als Lösung A2 bezeichnet. Der pH-Wert der Lösungen A1 und A2 betrug 11,0.

Die frisch angesetzte Lösung A1 enthielt voluminöse Abscheidungen in Schwebe, nach 24 h sanken die Abscheidungen zu Boden und oberhalb des Bodensatzes war eine feindisperse Emulsion sichtbar. Die frisch angesetzte Lösung A2 war eine feindisperse Emulsion, nach 24 h blieb die Emulsion feindispers, jedoch trat ein schwacher, feiner Bodensatz auf.

Nach 24 h wurden die gereiften Lösungen A1 und A2 mit Falternfiltern (Macherey Nagel 615 ¼) filtriert. Die Rückstände (Kalkseifen) im Filter wurden 2 h lang bei 105°C getrocknet und gravimetrisch analysiert.

| | **Mischung A1 (Vergleich)** | **Mischung A2 (erfindungsgemäß)** |
|---|---|---|
| **Masse des Rückstands (Kalkseifen)** | 0,89 g | 0,36 g |
| **Einwaage der Mischung** | 1,50 g | 1,50 g |
| **Verhältnis aus Masse des Rückstands zu Einwaage der Mischung** | 59 Gew.-% | 24 Gew.-% |

Bei der Mischung A1 betrug die Masse des Rückstands 0,89 g. Bei der erfindungsgemäßen Mischung A2 betrug die Masse des Rückstands lediglich 0,36 g.

### Fazit:

Mit den erfindungsgemäßen Beispielen konnte deutlich herausgestellt werden, dass bei der Verwendung des erfindungsgemäßen Produkts (Mischung A2) die Kalkseifenabscheidungen maßgeblich gemindert werden konnten.

Der Versuch zeigt, dass die Aufgabe der vorliegenden Erfindung erfüllt werden konnte. Die Mischung A1 wurde nach ausreichender Reaktionszeit so modifiziert, dass ein Produkt (Mischung A2) bereitgestellt wurde, welches ohne Emulgatorzusatz wassermischbar ist, eine starke Polarität besitzt, keine oder keine maßgebliche Neigung zur Bildung von Kalkseifenabscheidungen in hartem Wasser hat und einen hohen Schwefelgehalt aufweist.

## Patentansprüche

1. Schwefelbrückenhaltige Verbindungen, **dadurch gekennzeichnet, dass** innerhalb des Moleküls mindestens eine Fettsäure über mindestens eine Schwefelbrücke an mindestens einen Polyalkylenglykolester, dem Umsetzungsprodukt aus mindestens einer Fettsäure mit einem Polyalkylenglykol, verbunden ist und diese einen Schwefelgehalt von 8 bis 29 Gew.-% aufweisen.

2. Schwefelbrückenhaltige Verbindungen nach Anspruch 1, **dadurch gekennzeichnet, dass** es sich bei dem Polyalkylenglykolester um Polyethylenglykolester, Polypropylenglykolester, Polybutylenglykolester und/oder um Polyalkylenglykolester, bei denen die Ethylenoxid-, Propylenoxid- und/oder Butylenoxid-Einheiten statistisch und/oder blockweise angeordnet sind, vorzugsweise um Polyethylenglykolester, handelt.

3. Schwefelbrückenhaltige Verbindungen nach Anspruch 1 oder 2, **dadurch gekennzeichnet, dass** sie den folgenden Formeln entsprechen: 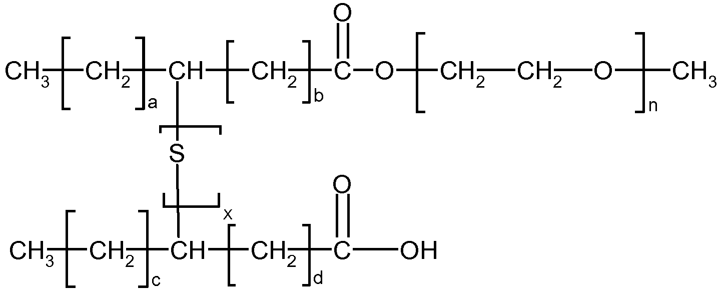 und/oder 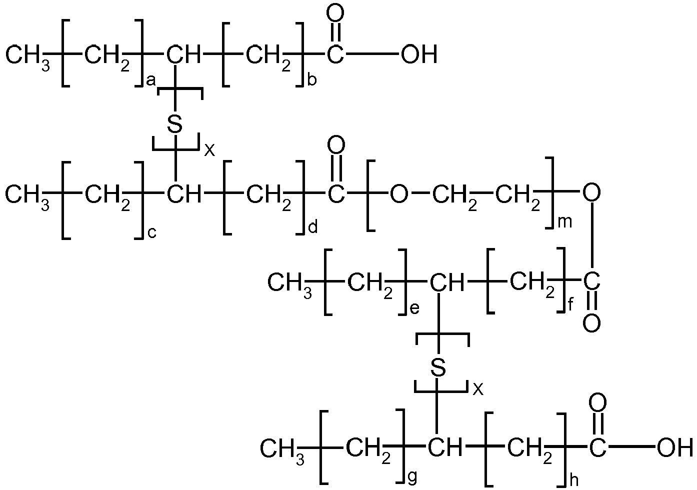 und/oder Formel (III) 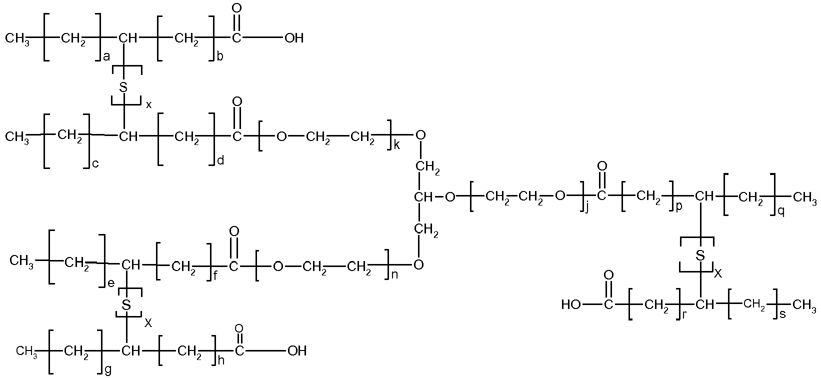 und /oder Formel (IV) 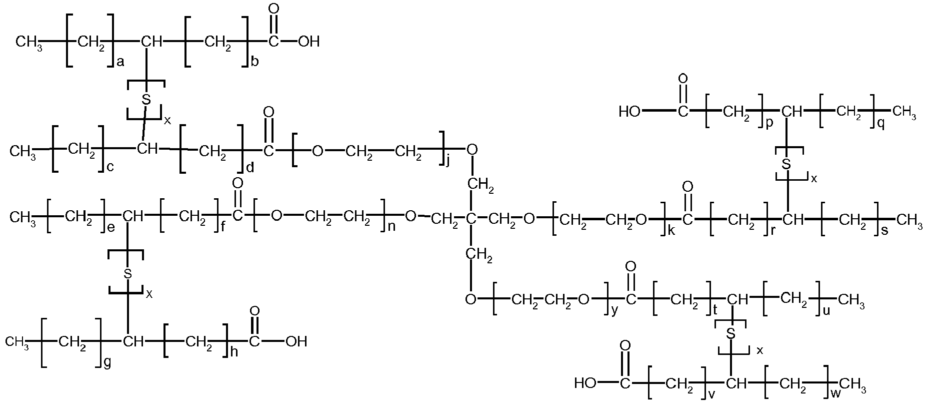
wobei a, b, c, d, e, f, g, h, p, q, r, s, t, u, v und w unabhängig voneinander Werten von 3 bis 29, vorzugsweise 5 bis 12 entsprechen und
n, k, j und y unabhängig voneinander Werten von 2 bis 10, vorzugsweise 4 bis 8 entsprechen, m = 4 bis 10, vorzugsweise 8 bis 16 ist,
x = 1 bis 10, vorzugsweise 2 bis 4 ist.

4. Mischungen enthaltend mindestens eine der schwefelbrückenhaltigen Verbindungen nach einem der Ansprüche 1 bis 3, und zusätzlich weitere Schmierstoffadditive und/oder Basisflüssigkeiten.

5. Verfahren zur Herstellung von schwefelbrückenhaltigen Verbindungen nach einem der Ansprüche 1 bis 4, **dadurch gekennzeichnet, dass** zuerst die ungesättigte Fettsäure, vorzugsweise die Monocarbonsäure, mit Schwefel und/oder Schwefelwasserstoff zur schwefelverbrückten Fettsäure umgesetzt wird und anschließend die Umsetzung dieser schwefelverbrückten Fettsäure mit Polyalkylenglykol erfolgt, so dass innerhalb der schwefelbrückenhaltigen Verbindungen mindestens eine Carbonsäureeinheit der Fettsäure enthalten ist.

6. Verfahren zur Herstellung von schwefelbrückenhaltigen Verbindungen nach einem der Ansprüche 1 bis 4, **dadurch gekennzeichnet, dass** zuerst ein molarer Überschuss an ungesättigter Fettsäure, vorzugsweise die ungesättigte Monocarbonsäure, mit Polyalkylenglykol zum Zwischenprodukt, bestehend aus ungesättigtem Polyalkylenglkyolester und ungesättgter Fettsäure, umgesetzt wird und anschließend die Umsetzung dieses Zwischenprodukts mit Schwefel und/oder Schwefelwasserstoff erfolgt, so dass innerhalb der schwefelbrückenhaltigen Verbindungen mindestens eine Carbonsäureeinheit der Fettsäure enthalten ist.

7. Verfahren zur Herstellung von schwefelbrückenhaltigen Verbindungen nach einem der Ansprüche 1 bis 4, **dadurch gekennzeichnet, dass** zuerst die ungesättigte Fettsäure, vorzugsweise ungesättigte Monocarbonsäure, mit mindestens einem ungesättigten Fettsäurepolyalkylenglykolester gemischt wird und anschließend die Umsetzung dieses Gemisches mit Schwefel und/oder Schwefelwasserstoff erfolgt, so dass innerhalb der erfindungsgemäßen schwefelbrückenhaltigen Verbindungen mindestens eine Carbonsäureeinheit der Fettsäure enthalten ist.

8. Verfahren nach Anspruch 5 bis 7, **dadurch gekennzeichnet, dass** als ungesättigte Fettsäuren, vorzugsweise ungesättigte Monocarbonsäuren mit einer Kohlenstoffkettenlänge von 6 bis 32 C-Atomen, und/oder Gemische aus ungesättigten Fettsäuren, vorzugsweise ungesättigten Monocarbonsäuren eingesetzt werden, welche als Nebenbestandteil gesättigte Fettsäuren enthalten können.

9. Verfahren nach einem der Ansprüche 5 bis 8, **dadurch gekennzeichnet, dass** der Schwefel in elementarer Form und/oder als Schwefelwasserstoff zugesetzt wird.

10. Verfahren nach einem der Ansprüche 5 bis 9, **dadurch gekennzeichnet, dass** als Polyalkylenglykol bifunktionelles Polyethylenglykol eingesetzt wird.

11. Verwendung der schwefelbrückenhaltigen Verbindungen nach einem der Ansprüche 1 bis 4 als Schwefelträger und/oder Schmierstoffadditiv.

## Claims

1. Sulphur-bridged compounds, **characterized in that** within the molecule there is at least one fatty acid bonded by way of at least one sulphur bridge to at least one polyalkylene glycol ester, the reaction product of at least one fatty acid with a polyalkylene glycol, and these have from 8 to 29% by weight sulphur content.

2. Sulphur-bridged compounds according to Claim 1, **characterized in that** the polyalkylene glycol ester is polyethylene glycol ester, polypropylene glycol ester, polybutylene glycol ester and/or polyalkylene glycol esters in which the ethylene oxide units, propylene oxide units and/or butylene oxide units are in random and/or blockwise arrangement, preferably being polyethylene glycol ester.

3. Sulphur-bridged compounds according to Claim 1 or 2, **characterized in that** they comply with the following formulae: 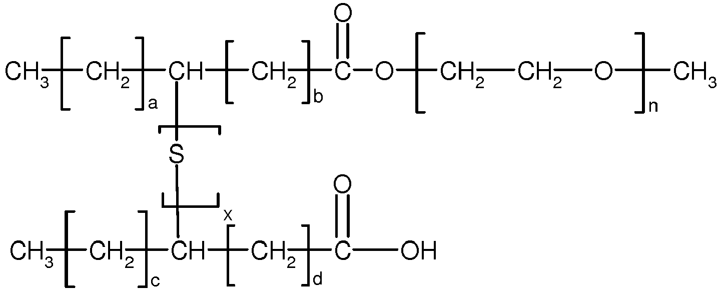 and/or 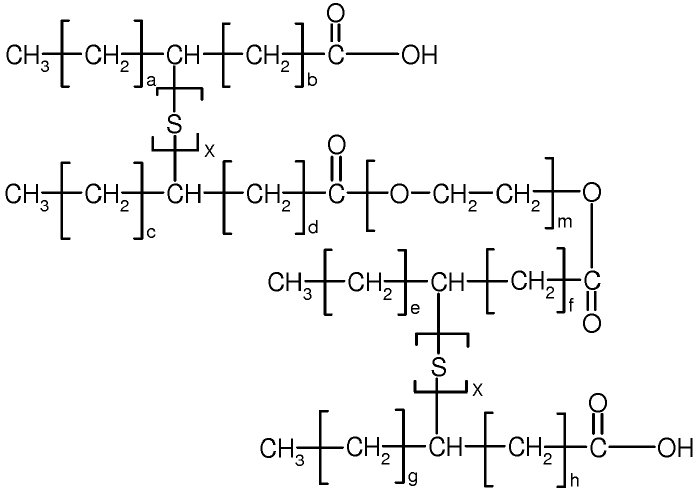 and/or formula (III) 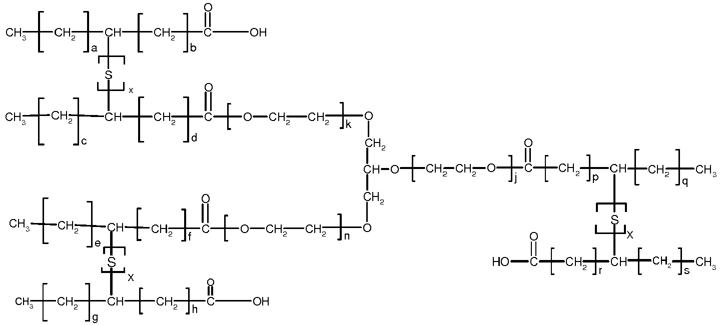 and/or formula (IV) 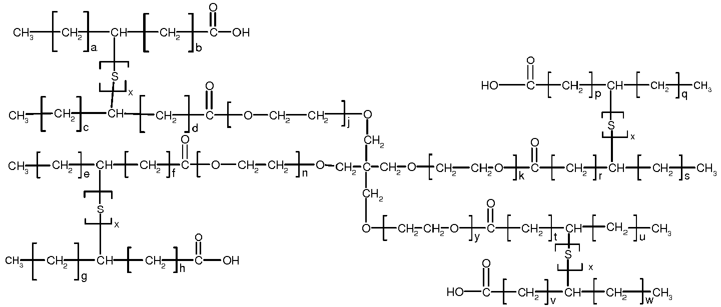
where a, b, c, d, e, f, g, h, p, q, r, s, t, u, v and w correspond mutually independently to values of from 3 to 29, preferably from 5 to 12 and
n, k, j and y correspond mutually independently to values of from 2 to 10, preferably from 4 to 8,
m = from 4 to 10, preferably from 8 to 16,
x = from 1 to 10, preferably from 2 to 4.

4. Mixtures comprising at least one of the sulphur-bridged compounds according to any of Claims 1 to 3, and additionally further lubricant additives and/or carrier liquids.

5. Process for the production of sulphur-bridged compounds according to any of Claims 1 to 4, **characterized in that** the unsaturated fatty acid, preferably the monocarboxylic acid, is first reacted with sulphur and/or hydrogen sulphide to give the sulphur-bridged fatty acid and then this sulphur-bridged fatty acid is reacted with polyalkylene glycol in such a way that at least one carboxylic acid unit of the fatty acid is present within the sulphur-bridged compounds.

6. Process for the production of sulphur-bridged compounds according to any of Claims 1 to 4, **characterized in that** a molar excess of unsaturated fatty acid, preferably the unsaturated monocarboxylic acid, is first reacted with polyalkylene glycol to give the intermediate product composed of unsaturated polyalkylene glycol ester and unsaturated fatty acid, and then this intermediate product is reacted with sulphur and/or hydrogen sulphide in such a way that at least one carboxylic acid unit of the fatty acid is present within the sulphur-bridged compounds.

7. Process for the production of sulphur-bridged compounds according to any of Claims 1 to 4, **characterized in that** the unsaturated fatty acid, preferably unsaturated monocarboxylic acid, is first mixed with at least one unsaturated fatty acid polyalkylene glycol ester and then this mixture is reacted with sulphur and/or hydrogen sulphide in such a way that at least one carboxylic acid unit of the fatty acid is present within the sulphur-bridged compounds of the invention.

8. Process according to any of Claims 5 to 7, **characterized in that** preferred unsaturated fatty acids used are unsaturated monocarboxylic acids having a carbon-chain length of from 6 to 32 C atoms, and/or mixtures of unsaturated fatty acids, preferably unsaturated monocarboxylic acids, where these can comprise saturated fatty acids as ancillary constituent.

9. Process according to any of Claims 5 to 8, **characterized in that** the sulphur is added in elemental form and/or as hydrogen sulphide.

10. Process according to any of Claims 5 to 9, **characterized in that** polyalkylene glycol used comprises bifunctional polyethylene glycol.

11. Use of the sulphur-bridged compounds according to any of Claims 1 to 4 as sulphur carrier and/or lubricant additive.

## Revendications

1. Composés contenant des ponts soufrés, **caractérisés en ce qu'**au moins un acide gras est relié par au moins un pont soufré à au moins un ester de polyalkylène glycol, le produit de réaction d'au moins un acide gras avec un polyalkylène glycol, dans la molécule, et ceux-ci présentent une teneur en soufre de 8 à 29 % en poids.

2. Composés contenant des ponts soufrés selon la revendication 1, **caractérisés en ce que** l'ester de polyalkylène glycol est un ester de polyéthylène glycol, un ester de polypropylène glycol, un ester de polybutylène glycol et/ou un ester de polyalkylène glycol dans lequel les unités oxyde d'éthylène, oxyde de propylène et/ou oxyde de butylène sont agencées statistiquement et/ou séquentiellement, de préférence un ester de polyéthylène glycol.

3. Composés contenant des ponts soufrés selon la revendication 1 ou 2, **caractérisés en ce qu'**ils correspondent aux formules suivantes : 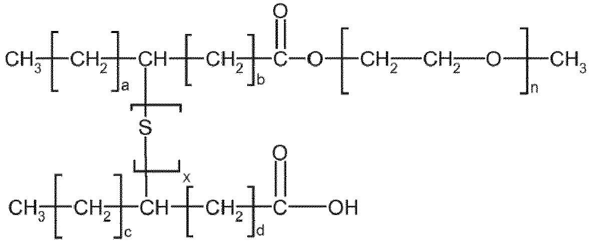 et/ou 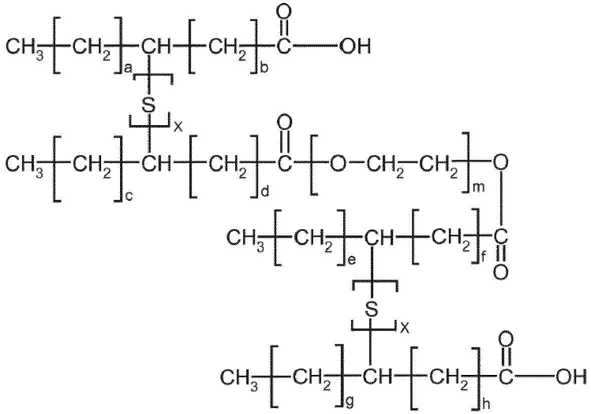 et/ou la formule (III) 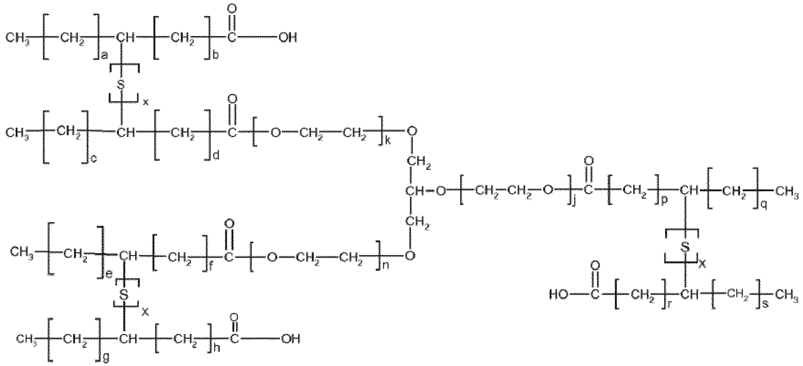 et/ou la formule (IV) 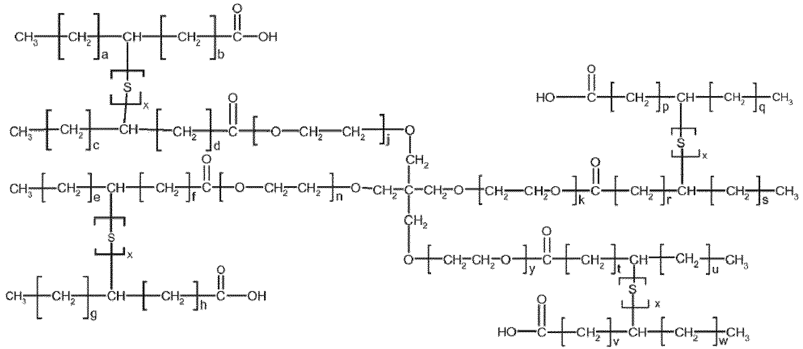
dans lesquelles a, b, c, d, e, f, g, h, p, q, r, s, t, u, v et w correspondent indépendamment les uns des autres à des valeurs de 3 à 29, de préférence de 5 à 12, et
n, k, j et y correspondent indépendamment les uns des autres à des valeurs de 2 à 10, de préférence de 4 à 8,
m = 4 à 10, de préférence 8 à 16,
x = 1 à 10, de préférence 2 à 4.

4. Mélanges contenant au moins un des composés contenant des ponts soufrés selon l'une quelconque des revendications 1 à 3, et en outre d'autres additifs lubrifiants et/ou liquides de base.

5. Procédé de fabrication de composés contenant des ponts soufrés selon l'une quelconque des revendications 1 à 4, **caractérisé en ce que** l'acide gras insaturé, de préférence l'acide monocarboxylique, est tout d'abord mis en réaction avec du soufre et/ou du sulfure d'hydrogène pour former l'acide gras ponté par du soufre, puis la réaction de cet acide gras ponté par du soufre avec un polyalkylène glycol a lieu, de sorte qu'au moins une unité acide carboxylique de l'acide gras soit contenue dans les composés contenant des ponts soufrés.

6. Procédé de fabrication de composés contenant des ponts soufrés selon l'une quelconque des revendications 1 à 4, **caractérisé en ce qu'**un excès molaire d'acide gras insaturé, de préférence l'acide monocarboxylique insaturé, est tout d'abord mis en réaction avec un polyalkylène glycol pour former le produit intermédiaire, constitué par l'ester de polyalkylène glycol insaturé et l'acide gras insaturé, puis la réaction de ce produit intermédiaire avec du soufre et/ou du sulfure d'hydrogène a lieu, de sorte qu'au moins une unité acide carboxylique de l'acide gras soit contenue dans les composés contenant des ponts soufrés.

7. Procédé de fabrication de composés contenant des ponts soufrés selon l'une quelconque des revendications 1 à 4, **caractérisé en ce que** l'acide gras insaturé, de préférence l'acide monocarboxylique insaturé, est tout d'abord mélangé avec au moins un ester de polyalkylène glycol d'acide gras insaturé, puis la réaction de ce mélange avec du soufre et/ou du sulfure d'hydrogène a lieu, de sorte qu'au moins une unité acide carboxylique de l'acide gras soit contenue dans les composés contenant des ponts soufrés selon l'invention.

8. Procédé selon les revendications 5 à 7, **caractérisé en ce que** des acides monocarboxyliques insaturés ayant une longueur de chaîne carbonée de 6 à 32 atomes C, et/ou des mélanges d'acides gras insaturés, de préférence d'acides monocarboxyliques insaturés, sont utilisés en tant qu'acides gras insaturés, qui peuvent contenir en tant que constituant secondaire des acides gras saturés.

9. Procédé selon l'une quelconque des revendications 5 à 8, **caractérisé en ce que** le soufre est ajouté sous forme élémentaire et/ou sous la forme de sulfure d'hydrogène.

10. Procédé selon l'une quelconque des revendications 5 à 9, **caractérisé en ce qu'**un polyéthylène glycol bifonctionnel est utilisé en tant que polyalkylène glycol.

11. Utilisation des composés contenant des ponts soufrés selon l'une quelconque des revendications 1 à 4 en tant que support de soufre et/ou additif lubrifiant.
